# EUROPEAN PATENT SPECIFICATION

(11) **EP 4 005 611 B1**
(45) Date of publication and mention of the grant of the patent: **26.11.2025**
(21) Application number: 20844930.6
(22) Date of filing: 22.07.2020
(51) Int. Cl.: A61M 1/16

(54) **DIALYSIS DEVICE**
DIALYSEGERÄT
DISPOSITIF DE DIALYSE

(30) Priority: 22.07.2019 JP 2019134512
(43) Date of publication of application: 01.06.2022
(73) Proprietor: School Juridical Person The Kitasato Institute, Tokyo 108-8641 (JP)
(72) Inventor: KOKUBO, Kenichi, Sagamihara-shi, Kanagawa 252-0373 (JP); KOBAYASHI, Kozue, Sagamihara-shi, Kanagawa 252-0373 (JP); KOBAYASHI, Hirosuke, Sagamihara-shi, Kanagawa 252-0373 (JP)
(74) Representative: Regimbeau
(86) International application number: PCT/JP2020/028429
(87) International publication number: WO 2021/015235

(56) References cited:
- JP-A- 2007 528 852
- US-A1- 2006 068 031
- US-A1- 2014 350 097
- US-A1- 2018 256 637
- US-A1- 2018 256 637
- US-A9- 2008 233 212

## Description

### Field of the Invention

The present invention relates to a dialyzer. Priority is claimed on Japanese Patent Application No. 2019-134512 filed July 22, 2019.

### Description of Related Art

Blood dialysis therapy is an essential life-supporting treatment for patients with end-stage renal disease. Examples of hemodialysis systems are disclosed in US 2018/0256637. However, it has been clarified that the blood dialysis therapy itself causes inflammation and weakened immunity in the patient's body, which causes disease complications in long-term dialysis patients. As one of the causes, side effects such as the activation of neutrophils, monocytes, or the like resulting from the contact of the patient's blood with material surfaces of a blood circuit and a dialysis membrane, which are originally foreign substances, increased oxidative stress resulting from the release of various cytokines and active oxygen, and the activation of platelets are considered.

In particular, since no special suppressing means have been taken at present against the activation of platelets, the activated platelet aggregates adhere to and aggregate on the material surfaces of the blood circuit and the dialysis membrane and then are peeled off by blood flow, and are transferred into a living body as the platelet aggregates. Among the platelet release factors released with the activation of platelets, it is considered that substances, which activate the platelets and leukocytes and promote arteriosclerosis, are present, and there is a possibility that long-term repeated platelet activation is involved in the disease complication of dialysis patients.

On the other hand, it is known that, in the living body, nitric oxide (hereinafter, may be referred to as "NO") is always released into the blood by nitric oxide synthase of vascular endothelial cells, and NO shows many actions such as the suppression of platelet aggregation and the suppression of adhesion of leukocytes, the suppression of migration and adhesion of the monocytes to the vascular endothelial cells (refer to, for example, Non-Patent Document 1). Additionally, it is also known that nitrite ions (hereinafter, may be referred to as "NO₂⁻") has the same actions as NO. Exemplary application of gaseous nitric oxide is disclosed in US 2008/0233212.

### Citation List

### [Non-Patent Document]

[Non-Patent Document 1]
Moro M. A., et al., CGMP mediates the vascular and platelet actions of nitric oxide: confirmation using an inhibitor of the soluble guanylyl cyclase., Proc Natl Acad Sci USA, 93 (4), 1480-1485, 1996.

### Summary of the Invention

### Technical Problem

An object of the present invention is to provide a dialyzer having few side effects.

### Solution to Problem

The invention is defined by the features of independent claim 1. The present invention includes the following aspects.
[1] A dialyzer including a dialysis unit including a blood channel and a dialysate channel; a dialysate supply channel configured to supply a dialysate to the dialysate channel; a dialysate discharge channel configured to discharge the dialysate from the dialysate channel; a blood supply channel configured to supply blood to the blood channel, and a blood discharge channel configured to discharge the blood from the blood channel, and the dialysate containing nitric oxide and/or nitrite ions, and the concentration of the nitric oxide being 0.5 to 10 µM or the concentration of the nitrite ions being 40 to 120 µM.
[2] The dialyzer in which the concentration of the nitric oxide contained in the dialysate is 0.5 to 6 µM, or the concentration of the nitrite ions contained in the dialysate is 50 to 100 µM.
[3] The dialyzer described in [1] or [2] in which the dialysis unit or the dialysate supply channel includes a material that releases the nitric oxide and/or the nitrite ions.
[4] The dialyzer according to any one of [1] to [3], further including a nitric oxide gas supply source, a flow meter connected to the nitric oxide gas supply source, and a nitric oxide gas supply path for supplying the nitric oxide gas to the dialysate.

### [Advantageous Effects of Invention]

According to the present invention, it is possible to provide a dialyzer having few side effects.

### Brief Description of the Drawings

Fig. 1 is a schematic diagram illustrating the structure of a dialyzer.
Fig. 2 is a schematic diagram illustrating the structure of the dialyzer.
Fig. 3 is a schematic diagram showing the structure of a circulation circuit used in Experimental Example 1.
Fig. 4 is a graph showing the 4-hour circulation achievement rate in Experimental Example 1.
Fig. 5 is a graph showing the measurement results of NO concentration in Experimental Example 1.
Fig. 6 is a graph showing the measurement results of a residual blood LDH activity value in Experimental Example 1.
Fig. 7 is a graph showing the measurement results of the NO concentration in a dialysate in contact with a column including NOC5-containing microcapsules in Experimental Example 2.
Fig. 8 is a graph showing the measurement results of the NO concentration in a dialysate in contact with a column including NOC12-containing microcapsules in Experimental Example 2.
Fig. 9 is a graph showing the measurement results of the residual blood LDH activity value in Experimental Example 3.

### Detailed Description of the Invention

Hereinafter, embodiments of the present invention will be described in detail referring the drawings according to the circumstances. In addition, in the drawings, the same or corresponding portions are designated by the same or corresponding reference numerals, and duplicated description will be omitted. In addition, there are portions in which the dimensional ratio in each figure is exaggerated for the sake of explanation and does not necessarily match the actual dimensional ratio.

### (First Embodiment)

A dialyzer according to a first embodiment and according to the claimed invention includes a dialysis unit including a blood channel and a dialysate channel, a dialysate supply channel configured to supply a dialysate to the dialysate channel, a dialysate discharge channel configured to discharge the dialysate from the dialysate channel, a blood supply channel configured to supply blood to the blood channel, and a blood discharge channel configured to discharge the blood from the blood channel, the dialysate contains NO and/or NO₂⁻, the concentration of NO is 0.5 to 10 µM, or the concentration of NO₂⁻ is 40 to 120 µM. In the dialyzer of the present embodiment, the dialysate may contain only NO, may contain only NO₂⁻, or may contain NO and NO₂⁻.

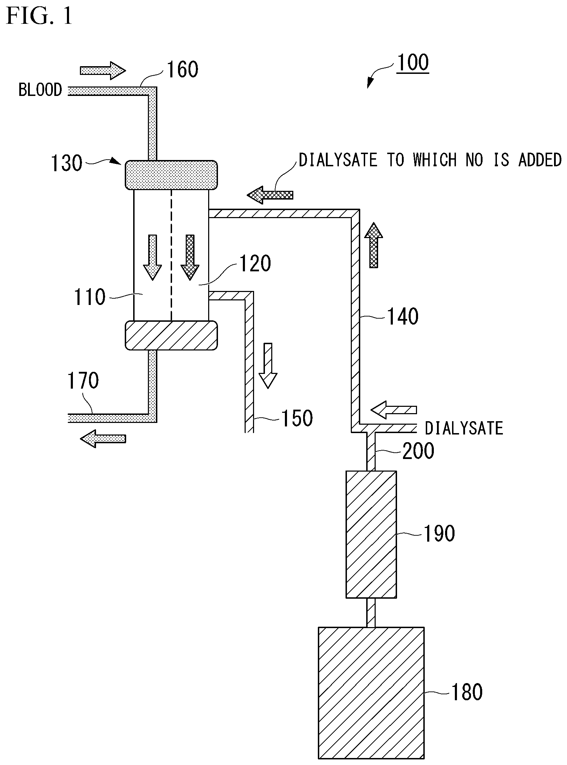

Fig. 1 is a schematic diagram illustrating the structure of the dialyzer of the first embodiment. As shown in Fig. 1, a dialyzer 100 includes a dialysis unit 130 including a blood channel 110 and a dialysate channel 120, a dialysate supply channel 140 for supplying a dialysate to the dialysate channel 120, a dialysate discharge channel 150 that discharges the dialysate from the dialysate channel 120, a blood supply channel 160 that supplies blood to the blood channel 110, and a blood discharge channel 170 that discharges the blood from the blood channel 110. In the dialyzer 100, the dialysate contains NO and/or NO₂⁻. The NO concentration contained in the dialysate is 0.5 to 10 µM, for example 0.5 to 6 µM, or for example 3 to 5 µM. Alternatively, the NO₂⁻concentration contained in the dialysate is 40 to 120 µM, for example, 50 to 100 µM, or for example, 60 to 90 µM.

As in the examples, the inventors has clarified that, when dialysis is performed by, a dialyzer in which the dialysate contains NO or NO₂⁻ and the concentration of NO or NO₂⁻ contained in the dialysate is in the above range, side effects such as blood coagulation are suppressed, no adverse events occur, and the dialysis treatment effect is high.

NO is hydrolyzed in water by the reactions represented by the following Formulas (1) to (5) and finally changed to NO₂⁻ and NO₃⁻.

· NO + 1/2O₂ → · NO₂ (1)

2·NO₂<=>N₂O₄ (2)

N₂N₄ + H₂O <=>NO₂⁻ + NO₃⁻ + 2H⁺ (3)

· NO + · NO₂ <=> N₂O₃ (4)

N₂O₃ + H₂O → 2NO₂- + 2H⁺ (5)

NO and NO₂⁻ show actions such as the suppression of platelet aggregation, the suppression of leukocyte adhesion, and the suppression of migration/adhesion of monocytes to vascular endothelial cells, but NO₃⁻ does not have such actions. For this reason, NO in the dialysate may be at least partially changed to NO₂⁻ or NO₃⁻, but is preferably in the form of NO or NO₂⁻.

Since it is difficult to directly measure the concentration of NO, it is simple and easy to completely hydrolyze NO and then measure the concentration as the total concentration of NO₂⁻ and NO₃⁻. In the present specification, the total concentration of NO and NO₂⁻ is measured as the total concentration of NO₂⁻ and NO₃⁻ after the NO is completely hydrolyzed.

A dialysis membrane such as a hollow fiber membrane is disposed inside the dialysis unit 130, and the blood channel 110 and the dialysate channel 120 are in contact with each other via the dialysis membrane. The blood flowing through the blood channel 110 and the dialysate flowing through the dialysate channel 120 exchange components, which can permeate the dialysis membrane, with each other inside the dialysis unit 130.

The means for containing the optimal concentration of NO and/or NO₂⁻ in the dialysate is not particularly limited. In the example of Fig. 1, the dialyzer 100 further includes a NO gas supply source 180, a flow meter 190 connected to the NO gas supply source 180, and a NO gas supply path 200 for supplying NO gas to the dialysate.

The NO gas supply path 200 is connected to the dialysate supply channel 140 and adds NO to the dialysate. Then, the dialysate to which NO is added flows through the dialysate supply channel 140 on the dialysis unit 130 side from a connection portion between the dialysate supply channel 140 and the NO gas supply path 200.

After that, the dialysate to which NO is added is in contact with the blood flowing through the blood channel 110 via the dialysis membrane inside the dialysis unit 130. Then, NO in the dialysate is added into the blood. The blood to which NO is added flows through the blood discharge channel 170 and is returned to a patient's body.

According to the dialyzer of the first embodiment, the biocompatibility of material surfaces of the blood channel and the dialysis membrane, which are originally foreign substances, is improved, and side effects such as a blood coagulation reaction in therapy in blood dialysis treatment and blood purification therapy conducted in an intensive treatment room (ICU) or the like are performed can be suppressed.

### (Second Embodiment)

A dialyzer according to a second embodiment includes a dialysis unit including a blood channel and a dialysate channel, a dialysate supply channel configured to supply a dialysate to the dialysate channel, a dialysate discharge channel configured to discharge the dialysate from the dialysate channel, a blood supply channel configured to supply blood to the blood channel, and a blood discharge channel configured to discharge the blood from the blood channel, the dialysate contains NO and/or NO₂⁻, the concentration of NO is 0.5 to 10 µM, or the concentration of NO₂⁻ is 40 to 120 µM. The dialysis unit or the dialysate supply channel includes a material that releases NO and/or NO₂⁻.

The material that releases NO and/or NO₂⁻ may be a material that releases only NO, may be a material that releases only NO₂⁻, or may be a material that releases NO and NO₂⁻. Examples of the material that releases NO and/or NO₂⁻ include columns containing NO donor-containing microcapsules, which will be described below in Examples.

The dialyzer of the second embodiment is mainly different from the dialyzer of the first embodiment in that the means for containing the optimal concentration of NO and/or NO₂⁻ in the dialysate contains a material that releases NO and/or NO₂⁻.

The dialyzer of the second embodiment may further include NO and/or NO₂⁻supply means other than the material that releases NO and/or NO₂⁻. Examples of the NO and/or NO₂⁻ supply means other than the material that releases NO and/or NO₂⁻includes a NO gas supply source, a flow meter connected to the NO gas supply source, a NO gas supply path for supplying NO gas to the dialysate, and the like, which are similar to those in the dialyzer of the first embodiment.

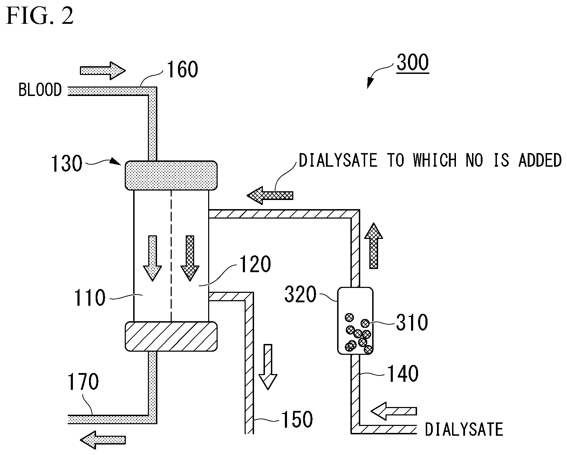

Fig. 2 is a schematic diagram illustrating the structure of the dialyzer of the second embodiment. As shown in Fig. 2, a dialyzer 300 includes a dialysis unit 130 including a blood channel 110 and a dialysate channel 120, a dialysate supply channel 140 for supplying a dialysate to the dialysate channel 120, a dialysate discharge channel 150 that discharges the dialysate from the dialysate channel 120, a blood supply channel 160 that supplies blood to the blood channel 110, and a blood discharge channel 170 that discharges the blood from the blood channel 110. The material that releases NO and/or NO₂⁻ is connected to the dialysate supply channel 140. In the example of Fig. 2, the material that releases NO and/or NO₂⁻ is a column 320 including NO donor-containing microcapsules 310 that release NO.

In the dialyzer 300, the concentrations of NO and NO₂⁻ in the dialysate are the same as those in the dialyzer of the first embodiment. The NO concentration contained in the dialysate is 0.5 to 10 µM, for example 0.5 to 6 µM, or for example 3 to 5 µM. Alternatively, the NO₂⁻ concentration contained in the dialysate is 40 to 120 µM, for example, 50 to 100 µM, or for example, 60 to 90 µM.

The material that releases NO and/or NO₂⁻ is not particularly limited, and is, for example, NO donors such as NOR1 (CAS No.: 163032-70-0, Half-life: 1.8 minutes), NOR3 (CAS No.: 138472-01-2, Half-life: 30 minutes), NOR4 (CAS No.: 162626-99-5, Half-life: 60 minutes), NOR5 (CAS No.: 174022-00-5, Half-life: 20 hours), NOC5 (CAS No.: 146724-82-5, Half-life: 25 minutes), NOC7 (CAS No.: 146724-84-7, Half-life: 5 minutes), NOC12 (CAS No.: 146724-89-2, Half-life: 100 minutes), NOC18 (CAS No.: 146724-94-9, Half-life: 21 hours); for example, catalytic materials that generate NO and/or NO₂⁻ from substances included in the living body, such as Cu-Pd catalysts and cyclone/Cu (II) catalysts; for example, NO₂⁻containing compounds such as sodium nitrite and calcium nitrite, and the like.

The microcapsules are capsules that have a core material and a wall material that covers the core material. The combination of the core material and the wall material can provide various functions such as improvement of stability, simplification of handleability, and impartation of sustained release.

In the dialyzer of the present embodiment, the method for producing NO and/or NO₂⁻ donor-containing microcapsules is not particularly limited, and the microcapsules may be produced by, for example, any method of chemical methods such as an interfacial polymerization method, a suspension polymerization method, a dispersion polymerization method, an in-situ polymerization method, an emulsion polymerization method, an in-liquid curing method; physicochemical methods such as an in-liquid drying method, a phase inversion emulsification method, a hetero-aggregation method, and a coacervation method; other methods such as a high-speed airflow impact method and a spray drying method.

By microencapsulating the NO and/or NO₂⁻ donor, the effects such as being possible to impart sustained release of NO and/or NO₂⁻ and maintain the supply of NO and/or NO₂⁻ for the time required for dialysis treatment and being simple and easy to adjust the concentration of NO and/or NO₂⁻ to be supplied to the dialysate are obtained.

In the dialyzer of the second embodiment, the material that releases NO and/or NO₂⁻ is connected to the dialysate supply channel 140, but such a material may be provided inside the dialysis unit 130.

### Examples

Hereinafter, the present invention will be described with reference to Examples, but the present invention is not limited to the following Examples. Animal experiments were conducted with the approval of the Animal Experiment Committee of the School of Health Sciences, Kitasato University (Approval Number: "Mamoru Ken 18-37").

### [Experimental Example 1]

### (Examination of indicated concentration of NO)

Blood dialysis of rats was performed by changing the amount of NO to be supplied to the dialysate, and the optimal concentration of NO was examined.

Male Sprague Dawley rats (body weight: 300 g to 400 g) that were 8 to 12 weeks old were used. The rats were inhaled and anesthetized using isoflurane 1.5% to 3.0% as an anesthetic. Blood was removed from the carotid artery, and blood was returned into the tail vein. The blood flow rate was 0.5 mL/min to 1.0 mL/min, the dialysate flow rate was 3 mL/min, and the blood dialysis was performed for 4 hours by a parallel flow operation. Heparin was administered at 0.7 units/g at the start of dialysis.

As the dialysis unit (dialyzer), a small rat dialyzer having a cellulose triacetate membrane, 140 hollow fibers, an effective length of 146 mm, and a membrane area of 0.014 m² were used. An extracorporeal perfusion type polymethylpentene membrane oxygenator was used for gas exchange.

NO gas of which the concentration was adjusted to 200 ppm, 400 ppm, and 800 ppm was added to the dialysate via a gas exchanger. As a control group, nitrogen gas (N₂) was added to the dialysate instead of NO gas.

During the experiment, arterial pressure, dialyzer inlet pressure, and venous pressure were measured at intervals of 15 minutes. Additionally, methemoglobin (Met-Hb) was measured at the start of the experiment and then at intervals of 60 minutes. Additionally, after the circulation was ended, the dialyzer was washed, and the amount of residual blood hemoglobin (Hb) amount and the activity value of residual blood lactate dehydrogenase (LDH) were measured.

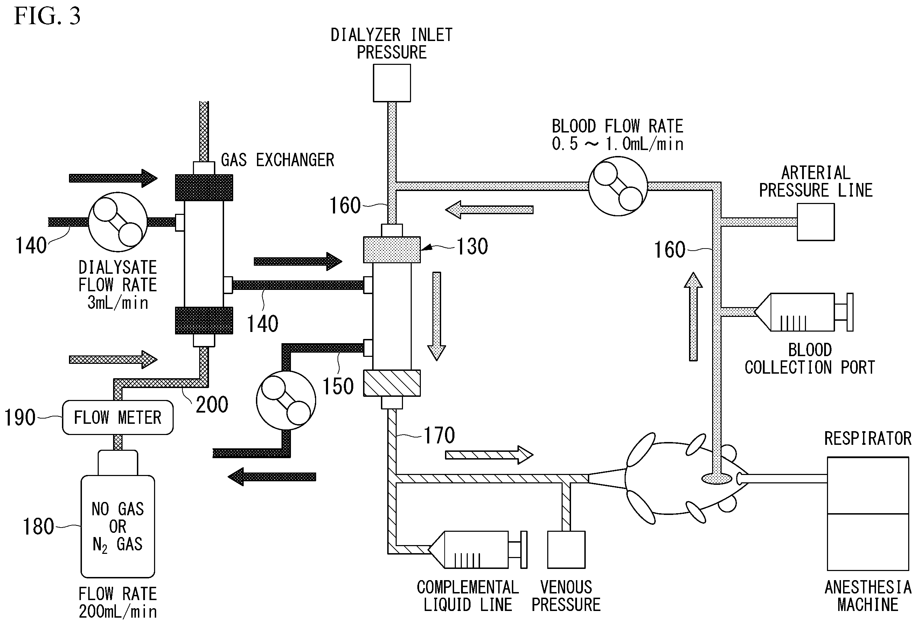

Fig. 3 is a schematic diagram showing the structure of a circulation circuit in which the dialyzer is connected to a rat. The experiment was performed with a circulation time of 4 hours. When the dialyzer inlet pressure or venous pressure exceeded 250 mmHg, the circulation was ended. In a case where it was possible to perform the 4-hours circulation, the residual blood in the dialyzer was evaluated.

### << Comparison of 4-hour circulation achievement rate >>

During the experiment, there was a case where the dialyzer inlet pressure or venous pressure increased, the blood coagulation in the dialyzer or the blood coagulation in the circuit were determined, and the circulation was stopped halfway. Thus, in all groups, the number of 4-hour circulation achievements with respect to the total number of experiments was calculated as the 4-hour circulation achievement rate.

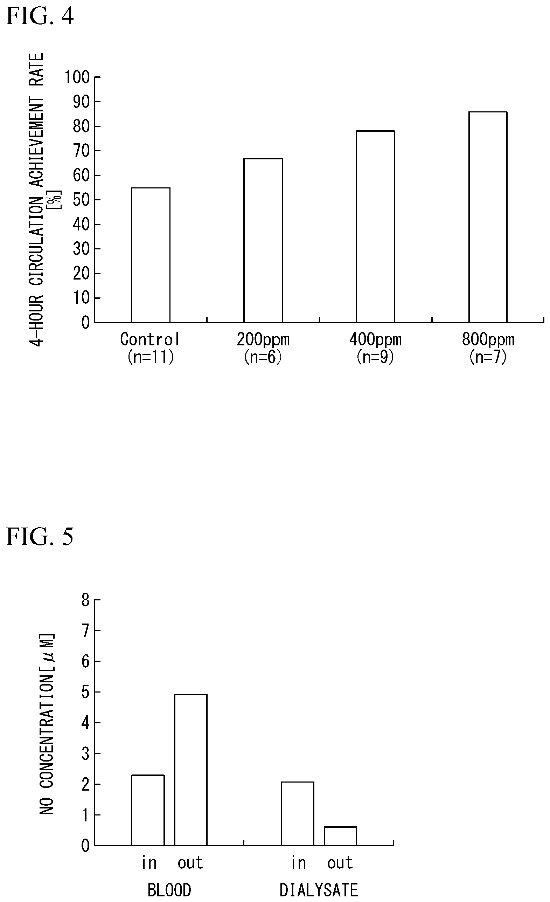

Fig. 4 is a graph showing the 4-hour circulation achievement rate. In Fig. 4, "Control" indicates the results of the control group, and "200 ppm", "400 ppm", and "800 ppm" indicate the results of a group in which NO gas of each concentration was supplied to the dialysate.

As a result, it was clarified that, as the concentration of NO gas to be supplied to the dialysate was higher, the 4-hour circulation achievement rate was increased.

### << Measurement of NO concentration >>

For a group in which 800 ppm of NO gas was supplied to the dialysate, the dialysate was supplied to the blood (in) on an arterial side of the dialysis unit, the blood (out) on a venous side of the dialysis unit, the dialysate (in) in the dialysate supply channel for supplying the dialysate to the dialysis unit, and the dialysate (out) in the dialysate discharge channel for discharging the dialysate from the dialysis unit were collected 1 hour after the start of circulation, and the NO concentration was measured using a commercially available kit ("OxiSelect In Vitro Nitric Oxide (Nitrite/Nitrate) Assay Kit (Colorimetric)" made by Cell Biolabs, Inc.). The NO concentration was measured as the sum of the concentrations of NO₂⁻ and NO₃⁻ after the NO was completely hydrolyzed. For this reason, hereinafter, there is a case where the total concentration of NO and NO₂⁻ is referred to as the NO concentration.

Fig. 5 is a graph showing the measurement results of the NO concentration. In addition, the NO concentration in blood at the start of circulation was 0 µM. As a result, it was clarified that the NO concentration in the dialysate in the dialysate supply channel was 2 µM in the group in which 800 ppm of NO gas was supplied to the dialysate. From the above result, the supplied "200 ppm" and "400 ppm" of NO gases are calculated to be 0.5 µM and 1 µM, each, in the dialysate.

Additionally, it was confirmed that the NO concentration in the blood (out) on the venous side of the dialysis unit is increased to be higher than the NO concentration in the blood (in) on the arterial side of the dialysis unit, and NO was added into the blood by the dialyzer. The amount of increase in NO concentration was about 3 µM. On the other hand, it was further supported that the NO concentration in the dialysate (out) in the dialysate discharge channel is decreased to be lower than the NO concentration in the dialysate (in) in the dialysate supply channel, and NO was added into the blood by the dialyzer. The amount of decrease in NO concentration was about 1 µM.

As described above, since the blood flow rate was 0.5 mL/min to 1.0 mL/min and the dialysate flow rate was 3 mL/min, the blood flow rate:dialysate flow rate = about 1:3. Thus, it was considered that the mass balance between the amount (about 3 µM) of increase in NO concentration in blood and the amount (about 1 µM) of decrease in NO concentration in dialysate was appropriate.

### << Measurement of residual blood LDH activity value after end of circulation >>

After the end of 4 hours of circulation, the dialyzer was washed with 10 mL of Lactec Injection and then a 0.5% Triton (Registered trademark) solution was circulated for 2 hours. After that, an LDH activity value in a washing solution was measured using a commercially available kit ("Cytotoxicity Measurement KitPLUS (LDH)" made by Roche Diagnostics K.K.) and used as the residual blood LDH activity value.

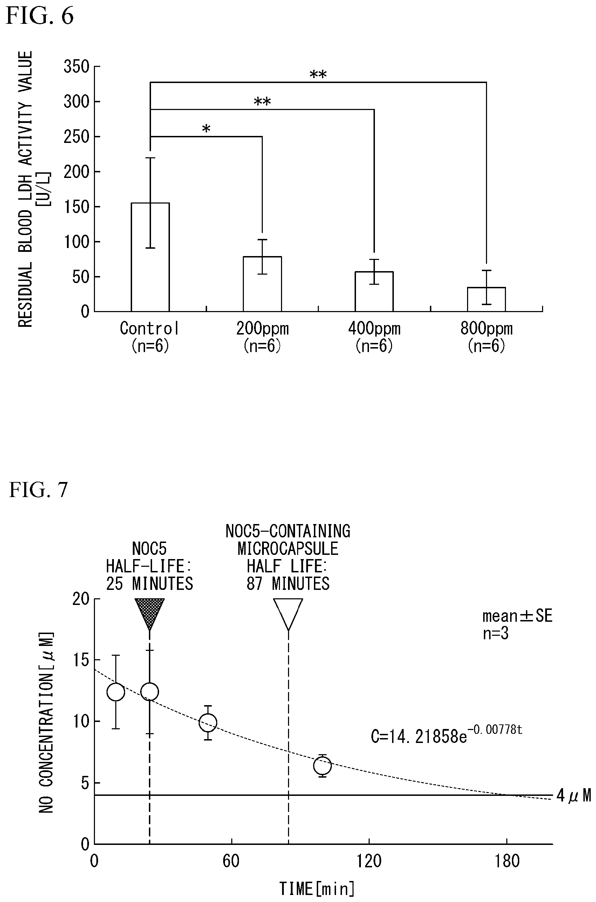

Fig. 6 is a graph showing the measurement results of the residual blood LDH activity value. In Fig. 6, "Control" indicates the results of the control group, and "200 ppm", "400 ppm", and "800 ppm" indicate the results of a group in which NO gas of each concentration was supplied to the dialysate. Additionally, "*" indicates that a significant difference is present at Tukey's test result p < 0.05, and "**" indicates that a significant difference is present at Tukey's test result p < 0.01.

As a result, it was clarified that the residual blood LDH activity value showed a significantly lower value in any of the groups to which 200 ppm, 400 ppm, and 800 ppm of NO gas were supplied to the dialysate as compared to the control group. Additionally, it was clarified that, as the NO concentration was higher, the residual blood LDH activity value showed a lower value. This result indicates that, as the amount of NO gas to be supplied to the dialysate is higher, there is the effect of suppressing the blood coagulation.

In addition, it was clarified that the amount of residual blood Hb was the same result as that of the residual blood LDH activity value, and the amount of residual blood Hb showed a significantly lower value in any of the groups in which 200 ppm, 400 ppm, and 800 ppm of NO gas were supplied to the dialysate as compared to the control group. Additionally, it was confirmed that, since Met-Hb was less than 10% in all NO-added groups, no adverse event occurred when NO gas having a concentration of at least 200 ppm to 800 ppm was supplied to the dialysate.

It was clarified that, since the 4-hour circulation achievement rate was higher at 800 ppm than at 200 ppm and 400 ppm, and Met-Hb did not exceed 10% even at 800 ppm, the blood coagulation could be reliably suppressed by supplying 800 ppm of NO gas to the dialysate.

By extrapolating the above results, when the NO concentration at which the 4-hour circulation achievement rate was 100% and the NO concentration at which the residual blood LDH activity value was 0 U/L were calculated, the NO concentrations were 5.4 µM and 6 µM.

### [Experimental Example 2]

### (Production of NO donor-containing microcapsule)

NO donor-containing microcapsules were produced. NOC5 (CAS No.: 146724-82-5, Half-life: 25 minutes) and NOC12 (CAS No.: 146724-89-2, Half-life: 100 minutes) were used as the NO donors.

First, 2 mL of chloroform and 10 mL of acetonitrile were added to 0.1 g of polylactic acid to completely dissolve the polylactic acid, and 1 mg of NO donor was further added and stirred well to prepare a dispersed phase. On the other hand, 0.1 g of soy-derived lecithin was dissolved in 190 mL of liquid paraffin to prepare a continuous phase.

Subsequently, the continuous phase and the dispersed phase were mixed with each other and placed in an egg-plant-shaped flask, and dried in liquid at 40°C for 3 hours under reduced pressure. After the drying in the liquid, the solution in the egg-plant-shaped flask was suction-filtered with a glass filter and washed with petroleum ether, and the formed microcapsules were recovered. The recovered microcapsules were in the form of fine white powder. Additionally, the amount of the recovered microcapsules was 0.0470 g to 0.1247 g.

### (Evaluation of NO donor-containing microcapsules)

The total amounts of the obtained NOC5-containing microcapsules and NOC12-containing microcapsules were packed in columns and connected to liquid feed tubes. Subsequently, the dialysate was fed at a flow rate of 1 mL/min, and the dialysate was brought into contact with the microcapsules.

For the NOC5 microcapsules, 2 mL of the dialysate was collected 10, 25, 50, and 100 minutes after the dialysate and the microcapsules came into contact with each other. For the NOC12 microcapsules, 2 mL of the dialysate was collected 10, 30, 50, 100, 150, and 180 minutes after the dialysate and the microcapsules came into contact with each other.

Subsequently, the NO concentration in each dialysate was measured using a commercially available kit ("OxiSelect In Vitro Nitric Oxide (Nitrite/Nitrate) Assay Kit (Colorimetric)" made by Cell Biolabs, Inc.). The NO concentration was measured as the sum of the concentrations of NO₂⁻ and NO₃⁻ after the NO was completely hydrolyzed.

Fig. 7 is a graph showing the measurement results of the NO concentration in the dialysate in contact with a column containing the NOC5-containing microcapsules. As a result, it was clarified that the NO concentration released from the NOC5-containing microcapsules decreased with the elapse of time. Approximate Formula C = 14.21858e^{-0.00778t} (here, C represents NO concentration [µM] and t represents time [minutes]) was obtained by exponential regression. From the obtained approximate formula, the NO concentration (initial concentration) at 0 minutes was calculated to be about 14 µM. Additionally, the half-life at which the initial concentration had a half value (about 7 µM) was calculated to be 87 minutes.

It was clarified that, since the half-life of the NO release amount of NOC5 was 25 minutes, the sustained release could be imparted by the microencapsulation. Additionally, the NO concentration in the dialysate could be maintained to be more than 4 µM for at least 100 minutes after the NOC5 microcapsules came into contact with the dialysate.

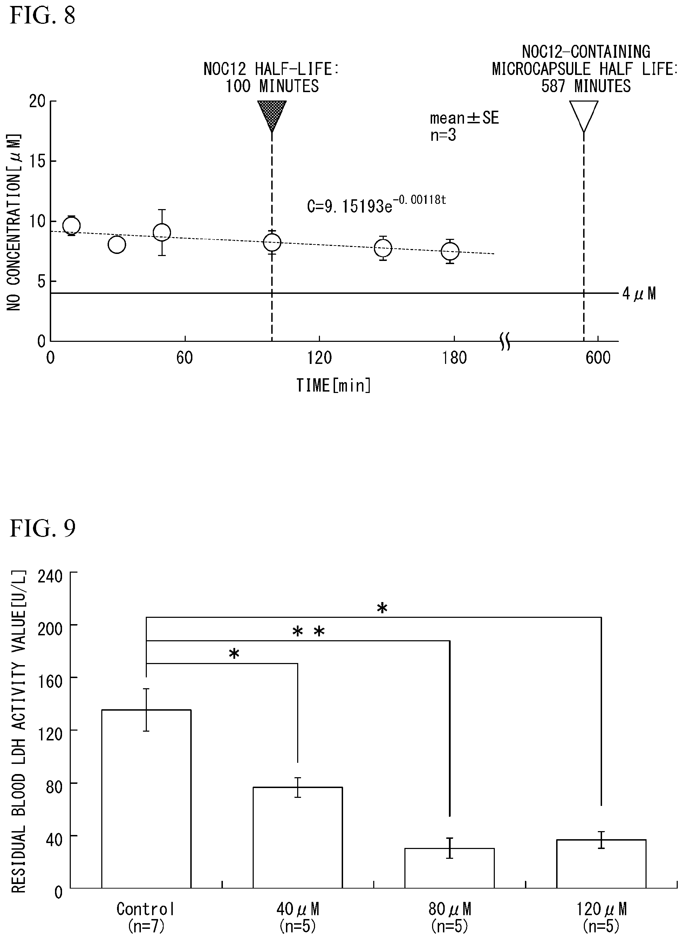

Fig. 8 is a graph showing the measurement results of the NO concentration in the dialysate in contact with a column containing the NOC12-containing microcapsules. As a result, it was clarified that the NO concentration released from the NOC12-containing microcapsules decreased with the elapse of time. Approximate Formula C = 9.15193e^{-0.00118t} (here, C represents NO concentration [µM] and t represents time [minutes]) was obtained by exponential regression. From the obtained approximate formula, the NO concentration (initial concentration) at 0 minutes was calculated to be about 9 µM. Additionally, the half-life at which the initial concentration had a half value (about 4.5 µM) was calculated to be 587 minutes.

It was clarified that, since the half-life of the NO release amount of NOC12 was 100 minutes, the sustained release could be imparted by the microencapsulation. Additionally, it was calculated that the NO concentration in the dialysate could be maintained to be more than 4 µM for 600 minutes after the NOC12 microcapsules came into contact with the dialysate.

### [Experimental Example 3]

### (Examination of optimal concentration of nitrite)

Blood dialysis of rats was performed by changing the amount of nitrite to be supplied to the dialysate, and the optimal concentration of nitrite was examined.

Male Sprague Dawley rats (body weight: 300 g to 400 g) that were 8 to 12 weeks old were used. The rats were inhaled and anesthetized using isoflurane 1.5% to 3.0% as an anesthetic. Blood was removed from the carotid artery, and blood was returned into the tail vein. The blood flow rate was 0.5 mL/min to 1.0 mL/min, the dialysate flow rate was 3 mL/min, and the blood dialysis was performed for 4 hours by a parallel flow operation. Heparin was administered at 0.7 units/g at the start of dialysis.

As the dialysis unit (dialyzer), a small rat dialyzer having a cellulose triacetate membrane, 140 hollow fibers, an effective length of 146 mm, and a membrane area of 0.014 m² were used.

Dialysates in which the sodium nitrite concentration was adjusted to be 40 µM, 80 µM, and 120 µM were used. As a control group, a dialysate containing no sodium nitrite was used.

During the experiment, arterial pressure, dialyzer inlet pressure, and venous pressure were measured at intervals of 15 minutes. Additionally, methemoglobin (Met-Hb) was measured at the start of the experiment and then at intervals of 60 minutes. Additionally, after the circulation was ended, the dialyzer was washed, and the amount of residual blood hemoglobin (Hb) amount and the activity value of residual blood lactate dehydrogenase (LDH) were measured.

The experiment was performed with a circulation time of 4 hours. When the dialyzer inlet pressure or venous pressure exceeded 250 mmHg, the circulation was ended. Both groups were allowed to circulate stably for 4 hours. The blood pressure (arterial pressure) during circulation was stable for 4 hours in 40 µM and 80 µM sodium nitrite-added groups, similar to the control group. However, in a 120 µM sodium nitrite-added group, the blood pressure is significantly lower than in the control group. In the 120 µM group, it is considered that vascular relaxation of peripheral blood vessels occurred and the blood pressure decreased.

The Met-Hb concentration had a value of 2% or less in all groups and was less than 10% referred to as a harmful range.

After the end of 4 hours of circulation, the dialyzer was washed with 10 mL of Lactec Injection and then a 0.5% Triton (Registered trademark) solution was circulated for 2 hours. After that, an LDH activity value in a washing solution was measured using a commercially available kit ("Cytotoxicity Measurement KitPLUS (LDH)" made by Roche Diagnostics K.K.) and used as the residual blood LDH activity value.

Fig. 9 is a graph showing the measurement results of the residual blood LDH activity value. In Fig. 9, "Control" indicates the results of the control group, and "40 µM", "80 µM", and "120 µM" indicate the results of a group in which each concentration of sodium nitrite was supplied to the dialysate. Additionally, "*" indicates that a significant difference is present at Tukey's test result p < 0.05, and "**" indicates that a significant difference is present at Tukey's test result p < 0.01.

As a result, it was clarified that the residual blood LDH activity value showed a significantly lower value in any of the groups in which 40 µM, 80 µM, and 120 µM of sodium nitrites were added to the dialysate as compared to the control group. Additionally, it was clarified that the residual blood LDH activity value showed a lower value at 80 µM and 120 µM as compared to 40 µM.

Regarding the amount of residual blood Hb, it was clarified that the amount of residual blood Hb showed a significantly lower value in the groups in which 80 µM and 120 µM sodium nitrites were added to the dialysate as compared to the control group.

It was clarified that, although the residual blood was less at 80 µM and 120 µM than at 40 µM, the blood coagulation could be particularly safely suppressed when nitrite ions were added such that the concentration of the nitrite ions was 50 to 100 µM because a slight decrease in blood pressure was observed at 120 µM and the Met-Hb did not exceed 10%.

### Industrial Applicability

According to the present invention, it is possible to provide a dialyzer having few side effects.

### Reference Signs List

100, 300: Dialyzer
110: Blood channel
120: Dialysate channel
130: Dialysis unit
140: Dialysate supply channel
150: Dialysate discharge channel
160: Blood supply channel
170: Blood discharge channel
180: NO gas supply source
190: Flow meter
200: NO gas supply path
310: NO donor-containing microcapsules
320: Column

## Claims

1. A dialyzer (100, 300) comprising:
a dialysis unit (130) including a blood channel (110) and a dialysate channel (120);
a dialysate supply channel (140) configured to supply a dialysate to the dialysate channel;
a dialysate discharge channel (150) configured to discharge the dialysate from the dialysate channel;
a blood supply channel (160) configured to supply blood to the blood channel, and
a blood discharge channel (170) configured to discharge the blood from the blood channel,
**characterized in that** the dialysate contains nitric oxide and/or nitrite ions, and a concentration of the nitric oxide is 0.5 to 10 µM or a concentration of the nitrite ions is 40 to 120 µM.

2. The dialyzer (100, 300) according to Claim 1,
wherein the concentration of the nitric oxide contained in the dialysate is 0.5 to 6 µM, or the concentration of the nitrite ions contained in the dialysate is 50 to 100 µM.

3. The dialyzer (100, 300) according to Claim 1 or 2,
wherein the dialysis unit (130) or the dialysate supply channel (140) includes a material that releases the nitric oxide and/or the nitrite ions.

4. The dialyzer (100, 300) according to any one of Claims 1 to 3, further comprising:
a nitric oxide gas supply source (180), a flow meter (190) connected to the nitric oxide gas supply source, and a nitric oxide gas supply path (200) for supplying the nitric oxide gas to the dialysate.

## Patentansprüche

1. Dialysator (100, 300), umfassend:
eine Dialyseeinheit (130), die einen Blutkanal (110) und einen Dialysatkanal (120) umfasst;
einen Dialysatzufuhrkanal (140), der dazu ausgestaltet ist, ein Dialysat zu dem Dialysatkanal zuzuführen;
einen Dialysatablasskanal (150), der dazu ausgestaltet ist, das Dialysat von dem Dialysatkanal abzulassen;
einen Blutzufuhrkanal (160), der dazu ausgestaltet ist, dem Blutkanal Blut zuzuführen; und
einen Blutablasskanal (170), der dazu ausgestaltet ist, das Blut von dem Blutkanal abzulassen,
**dadurch gekennzeichnet, dass** das Dialysat Stickoxid und/oder Nitrit-Ionen enthält und eine Konzentration des Stickoxids von 0,5 bis 10 µM beträgt oder eine Konzentration der Nitrit-Ionen von 40 bis 120 µM beträgt.

2. Dialysator (100, 300) nach Anspruch 1,
wobei die Konzentration des Stickoxids, das in dem Dialysat enthalten ist, von 0,5 bis 6 µM beträgt oder die Konzentration der Nitrit-Ionen, die in dem Dialysat enthalten sind, von 50 bis 100 µM beträgt.

3. Dialysator (100, 300) nach Anspruch 1 oder 2,
wobei die Dialyseeinheit (130) oder der Dialysatzufuhrkanal (140) ein Material umfasst, welches das Stickoxid und/oder die Nitrit-Ionen freisetzt.

4. Dialysator (100, 300) nach einem der Ansprüche 1 bis 3, ferner umfassend:
eine Stickoxid-Gaszufuhrquelle (180), einen Strömungsmesser (190), der mit der Stickoxid-Gaszufuhrquelle verbunden ist, und einen Stickoxid-Gaszufuhrweg (200) zum Zuführen des Stickoxidgases zu dem Dialysat.

## Revendications

1. Dialyseur (100, 300) comprenant :
une unité de dialyse (130) comprenant un canal sanguin (110) et un canal de dialysat (120) ;
un canal d'alimentation en dialysat (140) configuré pour alimenter le canal de dialysat en dialysat ;
un canal d'évacuation de dialysat (150) configuré pour évacuer le dialysat du canal de dialysat ;
un canal d'alimentation en sang (160) configuré pour alimenter le canal sanguin en sang, et
un canal d'évacuation de sang (170) configuré pour évacuer le sang du canal sanguin,
**caractérisé en ce que** le dialysat contient un oxyde nitrique et/ou des ions nitrite, et une concentration de l'oxyde nitrique est comprise entre 0,5 et 10 µM ou une concentration des ions nitrite est comprise entre 40 et 120 µM.

2. Dialyseur (100, 300) selon la revendication 1,
dans lequel la concentration en oxyde nitrique contenue dans le dialysat est comprise entre 0,5 et 6 µM, ou la concentration en ions nitrite contenue dans le dialysat est comprise entre 50 et 100 µM.

3. Dialyseur (100, 300) selon l'une quelconque des revendications 1 ou 2,
dans lequel l'unité de dialyse (130) ou le canal d'alimentation en dialysat (140) comprend un matériau qui libère l'oxyde nitrique et/ou les ions nitrite.

4. Dialyseur (100, 300) selon l'une quelconque des revendications 1 à 3, comprenant en outre :
une source d'alimentation en oxyde nitrique gazeux (180), un débitmètre (190) connecté à la source d'alimentation en oxyde nitrique gazeux, et un chemin d'alimentation en oxyde nitrique gazeux (200) pour fournir l'oxyde nitrique gazeux au dialysat.
